# EUROPEAN PATENT APPLICATION

(11) **EP 3 117 855 A1**
(43) Date of publication of application: **18.01.2017**
(21) Application number: 15176874.4
(22) Date of filing: 15.07.2015
(51) Int. Cl.: A61M 5/32

(54) **CAP AND CAP REMOVAL DEVICE**

(71) Applicant: Sanofi-Aventis Deutschland GmbH, 65929 Frankfurt am Main (DE)
(72) Inventor: The designation of the inventor has not yet been filed

(57) **Abstract**

The invention relates to a cap removal device (1) for removing and retaining a plurality of caps (7) from a drug delivery device (10), the cap removal device (1) comprising a body (2) having a plurality of recesses (4.1, 4.2, 4.3), wherein each of the plurality of recesses (4.1, 4.2, 4.3) comprises a shape corresponding to a shape of one of the plurality of caps (7) and wherein each recess (4.1, 4.2, 4.3) is provided with an engagement member (5.1, 5.2, 5.3) adapted to engage a cap (7). Furthermore, the invention relates to a cap (7) adapted to be mounted to a distal end of a drug delivery device (10), the cap (7) comprising a distal portion (7.2) comprising a shape corresponding to a shape of a recess (4.1, 4.2, 4.3) of the cap removal device (1).

## Description

### Technical Field

The invention relates to a cap for a drug delivery device and a cap removal device for removing the cap from the drug delivery device.

### Background of the Invention

WO 03/051423 A2 discloses a shield removal device for removing a shield from an assembly. A tubular member has a lower end and an upper end dimensioned to receive a shield. The tubular member includes an inner wall having at least one gripping element with a notch providing a flex point upon the gripping element. The flex point enables the gripping element to flex radially outwardly to receive the shield and permanently clasp the shield to withdraw the shield from the assembly.

There remains a need for an improved cap for a drug delivery device and for an improved cap removal device for removing the cap from the drug delivery device.

### Summary of the Invention

It is an object of the present invention to provide an improved cap for a drug delivery device and an improved cap removal device for removing the cap from the drug delivery device.

The object is achieved by a cap removal device according to claim 1 and by a cap according to claim 6.

Exemplary embodiments of the invention are given in the dependent claims.

According to the invention, a cap removal device for removing and retaining a plurality of caps from a drug delivery device comprises a body having a plurality of recesses, wherein each of the plurality of recesses comprises a shape corresponding to a shape of one of the plurality of caps and wherein each recess is provided with an engagement member adapted to engage one of the plurality of caps. In an exemplary embodiment the engagement member may engage the one of the plurality of caps form-fittingly and/or force-fittingly.

A cap according to the invention is adapted to be mounted to a distal end of a drug delivery device, the cap comprising a distal portion comprising a shape corresponding to a shape of the recess of the cap removal device.

The cap removal device according to the invention allows for facilitating removal of the cap for the user, in particular for users with impaired dexterity and preventing or reducing the risk of accidental removal of the cap. The cap removal device with the plurality of recesses allows for tracking a number of injections performed by counting the number of caps already held on the cap removal device. The cap removal device is compact and can be easily carried by a patient. The engagement between the cap and the recess also provides for improved safety, e.g. with a series of drug delivery device and/or injections. Furthermore, the engagement between a specific cap and a mating specific recess also provides for counterfeit protection.

In an exemplary embodiment, the shape of the recess comprises a circular cross section defining an axis. The distal portion of the cap may comprise a corresponding circular cross section. In other embodiments, the recess and the cap may comprise corresponding non-circular cross sections, e.g. elliptical, polygonal, triangular, or rectangular.

In an exemplary embodiment, the engagement member comprises one or more resilient latches arranged on a surface of the body or in side walls of the at least one recess, the one or more resilient latches protruding radially inwards, e.g. towards the axis, and adapted to engage within a circumferential notch on a radial surface of a cap, wherein the one or more resilient latches are adapted to be deflected by the cap upon insertion of the cap into the recess and allowed to relax into the circumferential notch thus latching the cap to the cap removal device. A radial surface of the cap may comprise one or more circumferential notches adapted to engage an engagement member of the cap removal device. The one or more circumferential notches may partially or completely extend around the radial surface. In an exemplary embodiment, the circumferential notch may comprise a plurality of circumferentially arranged recesses angularly spaced from each other.

A distal end of the distal portion of the cap may be adapted to deflect the one or more resilient latches on the cap removal device allowing the distal portion to enter the recess, wherein the circumferential notch is adapted to allow the one or more resilient latches to relax into the circumferential notch thus latching the cap to the cap removal device.

In order to remove the cap from a drug delivery device using the recess, the drug delivery device may be moved towards the cap removal device with the cap ahead, and the cap is inserted into the recess. A distal end of the distal portion deflects the resilient latches towards the body allowing the distal portion to enter the recess. Subsequently, the resilient latches are allowed to relax into the circumferential notch thus latching the cap to the cap removal device. As the drug delivery device is pulled away from the cap removal device, the cap remains latched to the cap removal device and is thus removed from the drug delivery device thus also removing a protective needle sheath from a needle of the drug delivery device, if applicable.

In an exemplary embodiment, the engagement member comprises one or more protrusions arranged on the surface or in side walls of the at least one recess, the one or more protrusions protruding radially inwards, e.g. towards the axis, and adapted to be angularly aligned with one or more cap recesses on a distal end of a cap to allow insertion of the cap into the recess in at least one angular position and to engage in a circumferential notch on a radial surface of the cap to allow the cap being rotated out of this rotational position relative to the cap removal device.

In an exemplary embodiment, the cap comprises one or more cap recesses on a distal end of the distal portion adapted to be angularly aligned with the one or more protrusions on the cap removal device allowing the one or more protrusions to pass through the one or more cap recesses into the circumferential notch, wherein the circumferential notch is adapted to allow rotation of the cap when the one or more protrusions are located in the circumferential notch for angularly misaligning the one or more protrusions with the one or more cap recesses.

In order to remove the cap from a drug delivery device using the recess, the drug delivery device is moved towards the cap removal device with the cap ahead and the cap is inserted into the recess. The one or more cap recesses are angularly aligned with the one or more protrusions such that upon insertion of the cap into the recess, the one or more protrusions pass through the one or more cap recesses into the circumferential notch. The drug delivery device and the cap may then be rotated clockwise or counter clockwise within the recess thus angularly misaligning the one or more protrusions with the one or more cap recesses such that the cap is held within the recess. As the drug delivery device is pulled away from the cap removal device, the cap remains latched to the cap removal device and is thus removed from the drug delivery device thus also removing the protective needle sheath from the needle, if applicable.

In an exemplary embodiment, the engagement member comprises one or more protrusions arranged on a ground surface of the at least one recess, the at least one protrusion adapted to be angularly aligned with at least one cap recess on a distal end of a cap to allow insertion of the cap into the recess in at least one angular position, wherein the at least one recess is dimensioned to hold one of the plurality of caps in a press fit and/or wherein the at least one protrusion is dimensioned to engage a cap recess of one of the plurality of caps in a press fit.

In an exemplary embodiment, the cap additionally comprises one or more cap recesses on a distal end of the distal portion adapted to be angularly aligned with one or more protrusions in the recess of the cap removal device, wherein the distal portion is dimensioned to press fittingly engage the recess and/or wherein the one or more cap recesses are dimensioned to press fittingly engage the one or more protrusions.

In order to remove the cap from a drug delivery device using the recess, the drug delivery device is moved towards the cap removal device with the cap ahead, and the cap is inserted into the recess. The one or more cap recesses are angularly aligned with the one or more protrusions such that upon insertion of the cap into the recess the one or more protrusions enter the one or more cap recesses. As the recess is dimensioned to hold the inserted cap in a press fit and/or the one or more protrusions are dimensioned to engage the one or more cap recesses in a press fit, the cap is held within the recess. As the drug delivery device is pulled away from the cap removal device, the cap remains latched to the cap removal device and is thus removed from the drug delivery device thus also removing the protective needle sheath from the needle, if applicable.

In an exemplary embodiment, the surface of the cap removal device in which the recesses are arranged is a top surface when the cap removal device is in an operating position. This allows the user to place the cap removal device onto a table or a similar surface and push the drug delivery device down onto the cap removal device in order to remove the cap without having to provide for a counteracting force, thus facilitating use.

In an exemplary embodiment, the cap may comprise a central bore adapted to receive and engage a protective needle sheath of an injection needle. The central bore may comprise one or more ledges or barbs or a friction fit to engage the protective needle sheath such that the protective needle sheath is removed from the needle when the cap is removed from the drug delivery device.

Furthermore, the present disclosure relates to a drug delivery device, comprising a cap mounted to a distal end of the drug delivery device.

Further scope of applicability of the present invention will become apparent from the detailed description given hereinafter. However, it should be understood that the detailed description and specific examples, while indicating exemplary embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic view of a drug delivery device with a cap,
- Figure 2: is a schematic transparent perspective view of a cap removal device for removing a cap from a drug delivery device,
- Figure 3: is a schematic top view of the cap removal device, and
- Figure 4: is a schematic perspective view of the cap removal device with two caps.

Corresponding parts are marked with the same reference symbols in all figures.

### Detailed Description

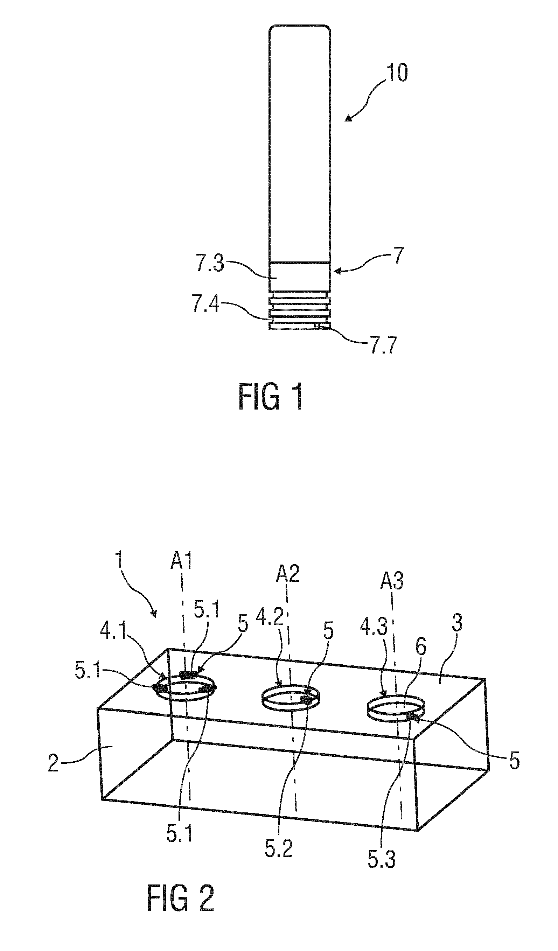

Figure 1 is a schematic view of a drug delivery device 10 with a cap 7 arranged at a distal end of the drug delivery device 10.

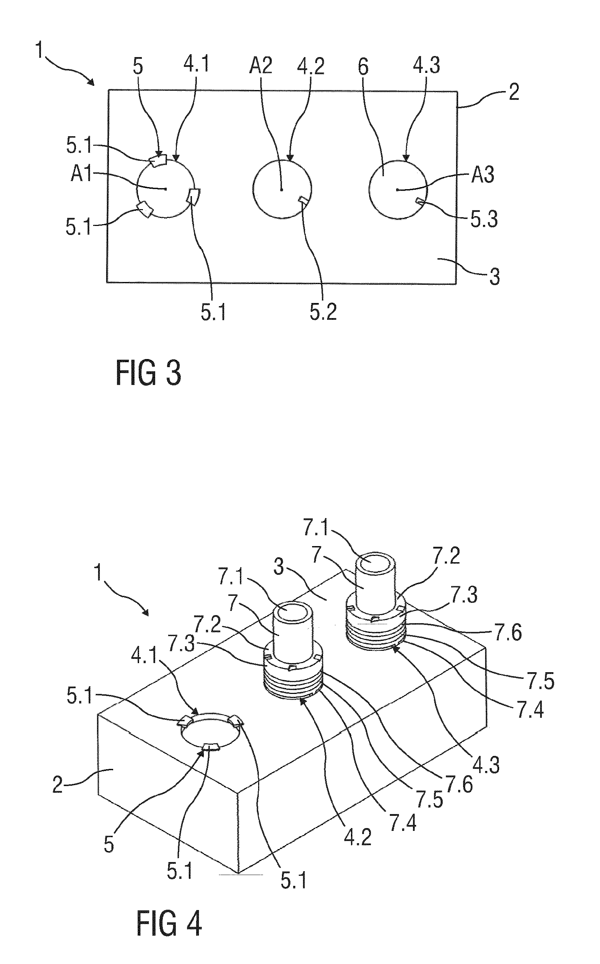

Figure 2 is a schematic transparent perspective view of a cap removal device 1 for removing a cap 7 from a drug delivery device 10. Figure 3 is a schematic top view of the cap removal device 1. The cap removal device 1 comprises a body 2 having a surface 3, in which one or more recesses 4.1, 4.2, 4.3 are provided. The recesses 4.1, 4.2, 4.3 respectively comprise a shape corresponding to a shape of a cap 7 of a drug delivery device 10. In an exemplary embodiment, the shape of the recess 4.1, 4.2, 4.3 comprises a circular cross section defining an axis A1, A2, A3. Each recess 4.1, 4.2, 4.3 is provided with an engagement member 5 adapted to engage the cap 7, e.g. a complementary engagement member on or in a cap 7.

In the illustrated embodiment, the engagement member 5 comprises one or more, e.g. three, resilient latches 5.1 arranged on the surface 3 or in side walls of the recess 4.1, the latches 5.1 protruding radially towards the axis A1 and adapted to engage within a circumferential notch 7.4 on a radial surface 7.3 of a cap 10.

The engagement member 5 of the recess 4.2 comprises one or more protrusions 5.2, e.g. one protrusion 5.2, arranged on the surface 3 or in side walls of the recess 4.2, the protrusion 5.2 protruding radially towards the axis A2 and adapted to be angularly aligned with a cap recess 7.7 on a distal end of a cap 7 to allow insertion of the cap 7 into the recess 4.2 in one angular position and then engage in a circumferential notch 7.4 on a radial surface 7.3 of the cap 7 to allow the cap 7 being rotated out of this rotational position.

The engagement member 5 of the recess 4.3 comprises one or more protrusions 5.3, e.g. one protrusion 5.3, arranged on a ground surface 6 of the recess 4.3, the protrusion adapted to be angularly aligned with a cap recess 7.7 on a distal end of a cap 7 to allow insertion of the cap 7 into the recess 4.3 in one angular position. The recess 4.3 is dimensioned to hold the inserted cap 7 in a press fit and/or the protrusions 5.3 is dimensioned to engage the cap recess 7.7 in a press fit.

Figure 4 is a schematic perspective view of the cap removal device 1 with two caps 7 held in the recesses 4.2 and 4.3. Each cap 7 is adapted to be mounted to a distal end of a drug delivery device 10 such as an injection device, e.g. an auto-injector, or an inhaler device. In an exemplary embodiment, the cap 7 comprises a central bore 7.1 adapted to receive and engage a protective needle sheath (not illustrated) arrangeable on an injection needle. For this purpose, the central bore 7.1 may comprise one or more ledges or barbs or a friction fit to engage the protective needle sheath such that upon removal of the cap 7 from a drug delivery device 10, the protective needle sheath is removed from the needle.

A distal portion 7.2 of the cap 7 comprises a shape corresponding to the shape of the recess 4.1, 4.2, 4.3 intended to engage the cap 7. In an exemplary embodiment, the distal portion 7.2 comprises a circular cross section. A radial surface 7.3 of the cap 7 may comprise one or more circumferential notches 7.4, 7.5, 7.6 which may partially or completely extend around the radial surface 7.3.

In order to remove the cap 7 from a drug delivery device 10 using the recess 4.1, the drug delivery device 10 is moved towards the cap removal device 1 with the cap 7 ahead and the cap 7 is inserted into the recess 4.1. A distal end of the distal portion 7.2 deflects the resilient latches 5.1 towards the body 2 allowing the distal portion 7.2 to enter the recess 4.1 and abut the ground surface 6. At this position, the resilient latches 5.1 are allowed to relax into the circumferential notch 7.4 thus latching the cap 7 to the cap removal device 1. As the drug delivery device 10 is pulled away from the cap removal device 1, the cap 7 remains latched to the cap removal device 1 and is thus removed from the drug delivery device 10, thus also removing the protective needle sheath from the needle, if applicable.

In order to remove the cap 7 from a drug delivery device 10 using the recess 4.2, the drug delivery device 10 is moved towards the cap removal device 1 with the cap 7 ahead and the cap 7 is inserted into the recess 4.2. In this case, the cap 7 additionally comprises one or more cap recesses 7.7 on a distal end of the distal portion 7.2 corresponding to the number of protrusions 5.2. The one or more cap recesses 7.7 are angularly aligned with the one or more protrusions 5.2 such that upon insertion of the cap 7 into the recess 4.2 the one or more protrusions 5.2 pass through the one or more cap recesses 7.7 into the circumferential notch 7.4. The drug delivery device 10 and the cap 7 may then be rotated clockwise or counter clockwise within the recess 4.2 thus angularly misaligning the one or more protrusions 5.2 with the one or more cap recesses 7.7 such that the cap 7 is held within the recess 4.2. As the drug delivery device 10 is pulled away from the cap removal device 1, the cap 7 remains latched to the cap removal device 1 and is thus removed from the drug delivery device 10, thus also removing the protective needle sheath from the needle, if applicable.

In order to remove the cap 7 from a drug delivery device 10 using the recess 4.3, the drug delivery device 10 is moved towards the cap removal device 1 with the cap 7 ahead and the cap 7 is inserted into the recess 4.3. In this case, the cap 7 comprises one or more cap recesses 7.7 on a distal end of the distal portion 7.2 corresponding to the number of protrusions 5.3. The one or more cap recesses 7.7 are angularly aligned with the one or more protrusions 5.3 such that upon insertion of the cap 7 into the recess 4.3, the one or more protrusions 5.3 enter the one or more cap recesses 7.7. As the recess 4.3 is dimensioned to hold the inserted cap 7 in a press fit and/or the one or more protrusions 5.3 are dimensioned to engage the one or more cap recesses 7.7 in a press fit, the cap 7 is held within the recess 4.3. As the drug delivery device 10 is pulled away from the cap removal device 1, the cap 7 remains latched to the cap removal device 1 and is thus removed from the drug delivery device 10, thus also removing the protective needle sheath from the needle, if applicable.

Those of skill in the art will understand that the cap removal device 1 may comprise any number of recesses 4.1, 4.2, 4.3 for engaging caps of drug delivery devices. In the illustrated embodiment, a cap removal device 1 with different types of engagement members 5 is shown by way of example. Those of skill in the art will understand that typically, the cap removal device 1 may comprise a plurality of recesses 4.1, 4.2, 4.3 with one and the same type of engagement member 5, for example only resilient latches 5.1 or only protrusions 5.2 or only protrusions 5.3.

The surface 3 may be a top surface when the cap removal device 1 is in an operating position.

The cap removal device 1 according to the invention allows for facilitating removal of the cap for the user, reducing the force required to remove the cap, preventing or reducing the risk of accidental removal of the cap and providing a counterfeit protection.

The cap removal device 1 with the plurality of recesses 4.1, 4.2, 4.3 allows for tracking a number of injections performed simply by counting the number of caps 7 already held on the cap removal device 1 even if the spent drug delivery device 10 has been discarded. The cap removal device 1 is compact and can be easily carried by a patient.

Those of skill in the art will understand that in alternative embodiments, the engagement member 5 may be arranged in a different way such that the cap 7 is insertable into the recess applying a force which may comprise a twisting or lever action, thus attaching the cap 7 to the cap removal device 1 by a form fit and/or by a friction fit. The cap 7 may be arranged to be screwed onto a drug delivery device 10. Likewise, the drug delivery device 10 or the cap 7 may comprise a lever facilitating removal of the cap 7. The shape on the cap removal device 1 and the corresponding shape on the cap 7 prevents a relative rotation, so the drug delivery device 10 can by unscrewed from the cap 7 while the cap 7 is held in the cap removal device 1. This decreases the force required for removing the cap 7 from the drug delivery device 10.

In an exemplary embodiment, a tracking unit may be arranged on the cap removal device 1 assisting a user in complying with a drug schedule or regimen. For example, numbers may be arranged next to each recess 4.1, 4.2, 4.3, allowing the user to track the number and order of injections.

The cap removal device 1 can be either disposable or reusable, i.e. disposed once the recesses 4.1, 4.2, 4.3 are filled or emptied and reused after the recesses 4.1, 4.2, 4.3 are filled.

In an exemplary embodiment, the number of injections may be tracked by having only one recess 4.1, 4.2, 4.3 that can be used multiple times. In this case the cap 7 may fall into a storage space underneath the recess 4.1, 4.2, 4.3, where the caps 7 may be stacked or stored side by side. The cap removal device 1 could have transparent walls or a window allowing the user to count the injections.

In an exemplary embodiment, the cap removal device 1 having one or a plurality of recesses 4.1, 4.2, 4.3 as described above, but integrated in a packaging for a plurality of drug delivery devices.

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a drug into a human or animal body. Without limitation, a drug delivery device may be an injection device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), implantable (e.g., coated stent, capsule), or feeding systems for the gastrointestinal tract. The presently described drugs may be particularly useful with injection devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.

Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia.

Exemplary DPP4 inhibitors are Vildagliptin, Sitagliptin, Denagliptin, Saxagliptin, Berberine.

Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')₂ fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

Those of skill in the art will understand that modifications (additions and/or removals) of various components of the substances, formulations, apparatuses, methods, systems and embodiments described herein may be made without departing from the full scope and spirit of the present invention, which encompass such modifications and any and all equivalents thereof.

### List of References

- 1: cap removal device
- 2: body
- 3: surface
- 4.1, 4.2, 4.3: recess
- 5: engagement member
- 5.1: resilient latch
- 5.2: protrusion
- 5.3: protrusion
- 6: ground surface
- 7: cap
- 7.1: central bore
- 7.2: distal portion
- 7.3: radial surface
- 7.4, 7.5, 7.6: circumferential notch
- 7.7: cap recess
- 10: drug delivery device
- A1, A2, A3: axis

## Claims

1. A cap removal device (1) for removing and retaining a plurality of caps (7) from a drug delivery device (10), the cap removal device (1) comprising a body (2) having a plurality of recesses (4.1, 4.2, 4.3), wherein each of the plurality of recesses (4.1, 4.2, 4.3) comprises a shape corresponding to a shape of one of the plurality of caps (7) and wherein each recess (4.1, 4.2, 4.3) is provided with an engagement member (5) adapted to engage one of the plurality of caps (7).

2. The cap removal device (1) according to claim 1, wherein the shape of the at least one recess (4.1, 4.2, 4.3) comprises a circular cross section.

3. The cap removal device (1) according to claim 1 or 2, wherein the engagement member (5) comprises one or more resilient latches (5.1) arranged on a surface (3) of the body (2) or in side walls of the at least one recess (4.1), the one or more resilient latches (5.1) protruding radially inwardsand adapted to engage within a circumferential notch (7.4) on a radial surface (7.3) of a cap (7), wherein the one or more resilient latches (5.1) are adapted to be deflected by the cap (7) upon insertion of the cap (7) into the recess (4.1) and allowed to relax into the circumferential notch (7.4) thus latching the cap (7) to the cap removal device (1).

4. The cap removal device (1) according to any one of the preceding claims, wherein the engagement member (5) comprises one or more protrusions (5.2) arranged on the surface (3) or in side walls of the at least one recess (4.2), the one or more protrusions (5.2) protruding radially inwardsand adapted to be angularly aligned with one or more cap recesses (7.7) on a distal end of a cap (7) to allow insertion of the cap (7) into the recess (4.2) in at least one angular position and to engage in a circumferential notch (7.4) on a radial surface (7.3) of the cap (7) to allow the cap (7) being rotated out of this rotational position relative to the cap removal device (1).

5. The cap removal device (1) according to any one of the preceding claims, wherein the engagement member (5) comprises one or more protrusions (5.3) arranged on a ground surface (6) of the at least one recess (4.3), the at least one protrusion (5.3) adapted to be angularly aligned with at least one cap recess (7.7) on a distal end of a cap (7) to allow insertion of the cap (7) into the recess (4.3) in at least one angular position, wherein the at least one recess (4.3) is dimensioned to hold one of the plurality of caps (7) in a press fit and/or wherein the at least one protrusion (5.3) is dimensioned to engage a cap recess (7.7) of one of the plurality of caps (7) in a press fit.

6. Cap (7) adapted to be mounted to a distal end of a drug delivery device (10), the cap (7) comprising a distal portion (7.2) comprising a shape corresponding to a shape of a recess (4.1, 4.2, 4.3) of the cap removal device (1) according to any one of the preceding claims.

7. Cap (7) according to claim 6, wherein the distal portion (7.2) comprises a circular cross section.

8. Cap (7) according to claim 6 or 7, wherein a radial surface (7.3) of the cap (7) comprises one or more circumferential notches (7.4, 7.5, 7.6) adapted to engage an engagement member (5) of the cap removal device (1).

9. Cap (7) according to claim 8, wherein the one or more circumferential notches (7.4, 7.5, 7.6) partially or completely extend around the radial surface (7.3).

10. Cap (7) according to claim 8 or 9, herein a distal end of the distal portion (7.2) is adapted to deflect one or more resilient latches (5.1) on the cap removal device (1) allowing the distal portion (7.2) to enter the recess (4.1), wherein the circumferential notch (7.4) is adapted to allow the one or more resilient latches (5.1) to relax into the circumferential notch (7.4) thus latching the cap (7) to the cap removal device (1).

11. Cap (7) according to claim 8 or 9, additionally comprising one or more cap recesses (7.7) on a distal end of the distal portion (7.2) adapted to be angularly aligned with one or more protrusions (5.2) on the cap removal device (1) allowing the one or more protrusions (5.2) to pass through the one or more cap recesses (7.7) into the circumferential notch (7.4), wherein the circumferential notch (7.4) is adapted to allow rotation of the cap (7) when the one or more protrusions (5.2) are in the circumferential notch (7.4) for angularly misaligning the one or more protrusions (5.2) with the one or more cap recesses (7.7).

12. Cap (7) according to any one of the claims 6 to 9, additionally comprising one or more cap recesses (7.7) on a distal end of the distal portion (7.2) adapted to be angularly aligned with one or more protrusions (5.3) in the recess (4.3) of the cap removal device (1), wherein the distal portion (7.2) is dimensioned to press fittingly engage the recess (4.3) and/or wherein the one or more cap recesses (7.7) are dimensioned to press fittingly engage the one or more protrusions (5.3).

13. Drug delivery device (10), comprising a cap (7) according to any one of the claims 6 to 12 mounted to a distal end of the drug delivery device (10).
